Europäisches Patentamt

**European Patent Office**

Office européen des brevets

(19)

(11) Numéro de publication: **0 005 296**
**B1**

(12) **FASCICULE DE BREVET EUROPEEN**

(45) Date de publication du fascicule du brevet: **06.01.82**

(51) Int. Cl.³: **C 07 C 19/02, C 07 C 17/10**

(21) Numéro de dépôt: **79200205.7**

(22) Date de dépôt: **02.05.79**

(54) **Procédé pour la fabrication de dichlorométhane.**

(30) Priorité: **05.05.78 FR 7813601**

(43) Date de publication de la demande:
**14.11.79 Bulletin 79/23**

(45) Mention de la délivrance du brevet:
**06.01.82 Bulletin 82/1**

(84) Etats contractants désignés:
**AT BE CH DE FR GB IT LU NL SE**

(56) Documents cités:
**FR - A - 816 990**
**FR - A - 894 906**
**FR - A - 2 081 871**
**FR - A - 2 263 213**
**US - A - 2 715 146**

(73) Titulaire: **SOLVAY & Cie (Société Anonyme)**
**Rue du Prince Albert, 33**
**B-1050 Bruxelles (BE)**

(72) Inventeur: **Forlano, Luigi**
**Rue Van Eyck, 50**
**B-1000 Bruxelles (BE)**
Inventeur: **Lerot, Luc**
**Rue de l'Equerre, 15**
**B-1140 Bruxelles (BE)**

(74) Mandataire: **Eischen, Roland**
**Solvay & Cie Département de la Propriété**
**Industrielle Rue de Ransbeek 310**
**B-1120 Bruxelles (BE)**

Courier Press, Leamington Spa, England.

# 0 005 296

## Procédé pour la fabrication de dichlorométhane

La présente invention concerne un procédé pour la fabrication du dichlorométhane par chloration du monochlorométhane.

Le dichlorométhane est fabriqué habituellement par chloration thermique du méthane ou du monochlorométhane. Ce procédé est peu sélectif car il donne le dichlorométhane sous forme d'un mélange avec des quantités importantes de produits plus chlorés, à savoir les tri- et tétra-chlorométhanes. En outre, il nécessite l'emploi de températures fort élevées et, de ce fait, il entraîne la formation de dépôts de carbone ou de goudrons sur les parois des réacteurs obligeant ainsi à des arrêts fréquents des installations.

Les brevets français FR—A—894906 et FR—A—816990 de leur côté décrivent respectivement des procédés de chloration de butane et de chlorure de méthyle en phase liquide et à l'intervention de rayonnements actiniques. Ces procédés présentent les inconvénients de nécessiter des appareillages particuliers soumis aux salissements des parties optiques et à des arrêts fréquents des installations. En outre, l'obtention de sélectivités acceptables en chlorure de méthylène requiert d'opérer à basse température.

Le brevet Etats-Unis US—A 2 715 146 décrit la fabrication de chlorure de méthylène par chloration du chlorure de méthyle en présence de monochlorure de soufre. Ce procédé présente l'inconvénient d'engendrer la formation de quantités relativement importantes de chloroforme, ce qui provoque une baisse de sélectivité en chlorure de méthylène.

La présente invention vise à fournir un procédé pour la fabrication de dichlorométhane qui ne présente pas les inconvénients des procédés conventionnels et qui permet notamment d'obtenir une haute productivité et une excellente sélectivité tout en étant réalisé à des températures facilement accessibles.

L'invention concerne à cet effet un procédé pour la fabrication de dichlorométhane par chloration du monochlorométhane en phase liquide en présence d'un initiateur de réactions radicalaires selon lequel on opère substantiellement en l'absence de chlore gazeux et en présence d'un initiateur de réactions radicalaires choisi parmi les composés peroxydés et les composés diazo.

Par "substantiellement en l'absence de chlore gazeux" on entend que moins de 10% et, de préférence moins de 5% en poids du chlore mis en oeuvre se trouve sous forme gazeuse, dans le mélange réactionnel à tout moment de la réaction. Les meilleurs résultats sont obtenus lorsque cette quantité est inférieure à 1% du poids total de chlore mis en oeuvre.

Le chlore peut avantageusement être mis en oeuvre sous forme de chlore liquide ou sous forme de chlore dissous dans du monochlorométhane liquide. La dissolution du chlore gazeux dans le monochlorométhane liquide étant une opération assez lente, on préfère, en général, pour des raisons économiques, mettre le chlore en oeuvre sous forme de chlore liquide.

Les quantités de chlore et de monochlorométhane mises en oeuvre sont choisies en général de manière que le rapport molaire chlore sur monochlorométhane soit inférieur à 1 et, de préférence, compris entre 0,1 et 0,5. Des rapports molaires supérieurs sont moins souhaitables car ils entraînent la formation de produits plus chlorés. Des rapports molaires inférieurs à 0,1 peuvent être utilisés, mais ils sont moins intéressants d'un point de vue économique car ils imposent de recycler des quantités importantes de produit non transformé.

Les initiateurs de réactions radicalaires utilisés dans le procédé selon la présente invention sont les composés peroxydés et les composés diazo, c'est-à-dire ceux comportant une fonction —N=N— dans leur molécule. On peut également utiliser des mélanges d'initiateurs.

En général, on utilise des initiateurs dont la constante de dissociation à 50°C dans le toluène est comprise entre $3.10^{-7}$ et $6.10^{-4}$ sec$^{-1}$ et, de préférence, entre $1.10^{-6}$ et $2.10^{-4}$ sec$^{-1}$.

De très bons résultats ont été obtenus avec les composés diazo qui sont donc particulièrement préférés. Comme composés diazo convenant bien en général, on peut citer:

le 2,2′-azo-bis-isobutyronitrile,
le 2,2′-azo-bis-2-méthylbutyronitrile,
le 2,2′-azo-bis-2-éthylpropionitrile,
le 1,1′-azo-bis-1-cyclopentanenitrile,
le 2,2′-azo-bis-2-cyclopropylpropionitrile,
le 2,2′-azo-bis-2,3-diméthylbutyronitrile,
le 2,2′-azo-bis-2-méthylvaléronitrile,
le 2,2′-azo-bis-2-cyclobutylpropionitrile,
le 2,2′-azo-bis-2-propylbutyronitrile,
le 2,2′-azo-bis-2-isopropylbutyronitrile,
le 2,2′-azo-bis-2-méthylhexylonitrile,
le 2,2′-azo-bis-2,3-diméthylvaleronitrile,
le 2,2′-azo-bis-2,4-diméthylvaleronitrile,
le 1,1′-azo-bis-1-cyclopentane-nitrile,

le 2,2'-azo-bis-2-méthylheptylonitrile,
le 2,2'-azo-bis-2-cyclopentylpropionitrile,
le 2,2'-azo-bis-2-cyclohexylpropionitrile,
le 2,2'-azo-bis-2-isopropyl-3-méthylbutyronitrile,
le 2,2'-azo-bis-2-benzylpropionitrile,
le 2,2'-azo-bis-2-isobutyl-4-méthylvaleronitrile,
le 2,2'-azo-bis-2-(4-chlorobenzyl)propionitrile,
le 2,2'-azo-bis-2-(4-nitrobenzyl)propionitrile,
le 1,1'-azo-bis-1-cyclododecanenitrile,
l'acide 4,4'-azo-bis-4-cyanopentanoique,
l'azo-bis-isobutyramidine,
le 2,2'-azo-bis-2-méthylpropionate de méthyle,
le 2,2'-azo-bis-isobutyrate de méthyle,
l'azo-bis-(N,N'-diméthylèneisobutyramidine),
l'azo-bis-(1-carbométhoxy-3-méthylpropane),
l'acide 2,2'-azo-bis-isobutyrique,
le 2,2'-azo-bis-2-méthylpropionate d'éthyle,
le 1,1'-azo-bis-1-chloro-1-phényléthane,
le 1,1'-azo-bis-1-chloro-1-(3-bromophényl)-éthane,
le 1,1'-azo-bis-1-chloro-1-(4-bromophényl)-éthane,
le 1,1'-azo-bis-1-chloro-1-(4-chlorophényl)-éthane,
le 3,7-diphényl-1,2-diaza-1-cycloheptène,
le 3,6-diphényl-1,2-diaza-1-cyclohexène,
le 1,1-azo-bis-cumène,
le 1,1'-azo-bis-3-chlorocumène,
le 1,1'-azo-bis-4-chlorocumène,
le 1,1'-azo-bis-4-fluorocumène,
le 1,1'-azo-bis-1-chloro-1-(4-tolyl)éthane,
le 2,2'-azo-bis-2-phényl-hexafluoropropane,
le 1,1'-azo-bis-1-acetoxy-1-phényléthane,
le 2,2'-azo-bis-2-(4-tolyl)-propane,
le phenyl-azo-triphénylméthane,
les 3- et 4-bromophényl-azo-triphénylméthane,
le 4-hydroxyphényl-azo-triphénylméthane,
les 2- et 4-nitrophényl-azo-9-phénylfluorène,
le 2,4-dinitrophényl-azo-9-phénylfluorène,
les 2-, 3- et 4-nitrophényl-azo-triphénylméthane,
le 2,4-dinitrophényl-azo-triphénylméthane,
l'azo-bis-diphénylméthane,
le 1,1'-azo-bis-1-(4-tolyl)-cyclohexane,
le 4-méthoxyphényl-azo-triphénylméthane,
les 3- et 4-tolyl-azo-triphénylméthane et
le 4-acetaminophényl-azo-triphénylméthane.

D'excellents résultats sont obtenus avec le 2,2'-azo-bis-2,4-diméthylvaléronitrile dont l'utilisation est donc tout particulièrement préférée.

La quantité d'initiateur mise en oeuvre peut varier dans d'assez larges limites; elle est en général comprise entre $1.10^{-2}$ et $2.10^{-6}$ et, de préférence, entre $5.10^{-3}$ et $10^{-5}$ mole par mole de chlore mis en oeuvre. Des concentrations supérieures peuvent convenir mais n'améliorent pas significativement les performances. Aux concentrations inférieures, la réaction est lente et on n'observe pas la transformation complète du chlore mis en oeuvre.

L'initiateur est en général mis en oeuvre soit tel quel, soit sous forme de solution dans un solvant organique. Dans ce cas, on utilise en général comme solvant un hydrocarbure chloré. De bons résultats ont été obtenus en utilisant pour ce faire un méthane chloré ou un mélange de méthanes chlorés. Le tétrachlorométhane convient bien à cette fin. Les quantités de solvant organique mises en oeuvre sont très variables mais en général elles sont telles que la concentration en solvant organique dans le mélange de départ ne dépasse pas 20% et de préférence 10% du poids total du mélange de départ. De préférence, le solvant organique est mis en oeuvre dans des proportions telles que sa concentration est comprise entre 0,01 et 10% du poids du mélange. Pour obtenir une productivité élevée des réacteurs, le solvant est habituellement utilisé en quantité égale ou légèrement supérieure à celle nécessaire pour dissoudre complètement la quantité d'initiateur mis en oeuvre.

En plus des réactifs, de l'initiateur et éventuellement de son solvant, de petites quantités d'autres constituants, ne dépassant en général pas 5% et, de préférence, 2% du poids du mélange réactionnel peuvent également être présentes dans celui-ci. Ces constituants peuvent être des sous-produits de la fabrication du monochlorométhane par chloration du méthane ou hydrochloration du méthanol.

3

La présence dans le mélange réactionnel de sels ferriques et d'oxygène provoque une diminution sensible du taux de transformation du chlore. On peut cependant tolérer de très petites quantités de ces inhibiteurs dans le mélange réactionnel. En général, la teneur en oxygène est maintenue inférieure à $10^{-3}$ et, de préférence, inférieure à $5.10^{-4}$ mole par mole de chlore et la teneur en ions ferriques est maintenue à un niveau inférieur à 100 ppm et, de préférence, inférieur à 15 ppm par rapport au poids de chlorure de méthyle.

La température de réaction est en général comprise entre 0 et 120°C et, de préférence, entre 15 et 80°C. La pression de réaction est suffisamment élevée pour maintenir le mélange réactionnel sous forme liquide. En général, on utilise des pressions comprises entre 0,8 et 100 kg/cm² et, le plus souvent, entre 2 et 70 kg/cm².

Le temps de séjour des réactifs dans la ou les zones réactionnelles peut varier dans d'assez larges limites. En général, on le choisit suffisamment long pour que tout le chlore soit transformé. Des temps de séjour compris entre 1 et 500 minutes et, de préférence, entre 2 et 200 minutes sont utilisés en général.

La réaction peut être réalisée dans une seule zone réactionnelle ou dans plusieurs zones réactionnelles successives. Si on utilise plusieurs zones réactionnelles successives, celles-ci peuvent être maintenues à une même température ou à des températures différentes les unes des autres. En général, la température de chacune des zones réactionnelles successives va en augmentant quand on passe de la première à la dernière zone; on peut ainsi assurer un taux de transformation quasi quantitatif du chlore.

Le monochlorométhane est en général introduit dans le mélange réactionnel en une seule fois au début de la réaction. Le chlore et l'initiateur peuvent faire l'objet d'une introduction unique ou d'une introduction étagée. L'introduction étagée de ces deux produits permet de contrôler la température du mélange réactionnel. L'introduction étagée de l'intitiateur permet en outre de compenser les pertes éventuelles d'initiateur et d'améliorer le taux de transformation du chlore.

Le procédé peut être réalisé en continu ou en discontinu. Industriellement, on utilise en général des réacteurs fonctionnant en continu. Ces réacteurs peuvent être de divers types connus en eux-mêmes. Ainsi, on peut opérer dans des réacteurs mélangeurs ou dans des réacteurs tubulaires dits "réacteurs méthodiques" ou "réacteurs intégraux". Lorsqu'on opère en discontinu, on utilise en général de réacteurs mélangeurs dans lesquels on introduit l'initiateur ainsi que le chlore et le monochlorométhane soit séparément soit sous forme de mélanges. Lorsqu'on opère en continu, on utilise de préférence plusieurs réacteurs mélangeurs disposés en cascade ou des réacteurs tubulaires. Selon un mode de réalisation préféré, on opère dans un réacteur tubulaire dans lequel le mélange réactionnel s'écoule et a une composition variable dans l'espace. Ce mode de réalisation permet d'obtenir une sélectivité particulièrement bonne.

Le dichlorométhane est utilisé dans de nombreuses applications et notamment comme agent propulseur et comme agent décapant des peintures.

Les exemples ci-après sont donnés afin de mettre en évidence les avantages du procédé selon l'invention par rapport aux procédés conventionnels.

Exemple 1

*Chloration thermique du monochlorométhane*

Cet exemple est donné à titre de comparaison.

L'essai consiste à introduire en continu dans un réacteur tubulaire maintenu à 550°C du chlore et du monochlorométhane mis en oeuvre dans un rapport molaire de 0,3. On obtient un mélange de méthanes chlorés ayant la composition ci-après:

| méthane chloré | % molaire |
|---|---|
| $CH_3Cl$ | 73,2 |
| $CH_2Cl_2$ | 23,79 |
| $CHCl_3$ | 2,92 |
| $CCl_4$ | 0,09 |

La sélectivité exprimée en dichlorométhane formé par rapport au monochlorométhane transformé est de 0,89 et le rapport molaire dichlorométhane sur la somme des méthanes plus chlorés est de 7,9.

Exemple 2

*Chloration initiée du monochlorométhane par du chlore gazeux*

Cet exemple est donné à titre de comparaison.

Le réacteur est constitué d'une cuve en acier de 1,5 litres pourvue d'un agitateur, d'un trop plein, d'introductions de chlore, de monochlorométhane, de catalyseur et de solvant et surmontée d'un condenseur servant à la récupération de produits gazeux. Le tétrachlorométhane est utilisé comme solvant de réaction.

La réaction est réalisée à 45°C. On introduit en continu dans le réacteur du chlore et du monochlorométhane gazeux et mis en oeuvre dans un rapport molaire égal à 0,3. Le peroxyde de lauroyle utilisé comme initiateur est mis en oeuvre à raison de $1.10^{-3}$ mole par mole de chlore.

Les produits issus du réacteur sont constitués de tétrachlorométhane, de monochlorométhane non transformé, de chlorure d'hydrogène et de chlorométhanes formés au cours de la réaction. Le tétrachlorométhane soutiré est recyclé au réacteur.

L'analyse des produits de la réaction a donné la composition suivante, le tétrachlorométhane formé étant estimé par analogie avec les résultats de la chloration thermique

| méthane chloré | % molaire |
|---|---|
| $CH_3Cl$ | 73,42 |
| $CH_2Cl_2$ | 23,25 |
| $CHCl_3$ | 3,21 |
| $CCl_4$ | $\sim 0,1$ |

La sélectivité en dichlorométhane formé par rapport au monochlorométhane transformé est de 0,87 et le rapport molaire dichlorométhane sur la somme des méthanes plus chlorés est de 7.

Exemples 3 à 5

*Chloration initiée du monochlorométhane par du chlore liquide*

Les exemples illustrent le procédé selon l'invention.

Les essais sont réalisés en discontinu dans un autoclave en acier inoxydable de 1,3 litres entouré d'une double paroi thermostatique et pourvu d'un agitateur.

On y introduit successivement le monochlorométhane liquide, le chlore liquide et enfin la solution d'initiateur sous pression d'azote. Les conditions opératoires et les résultats obtenus sont repris au Tableau I ci-après. Dans le tableau I $CH_3Cl_0$ représente le nombre de moles initial de monochlorométhane et $CH_3Cl_t$ le nombre de moles de monochlorométhane encore présentes après le temps t de réaction.

Dans tous les cas la quantité d'ions ferriques était inférieure à 15 mg Fe par litre.

Les essais 3 et 4 ont été réalisés avec du monochlorométhane de qualité pour analyse et l'essai 5 a été réalisé avec du monochlorométhane produit industriellement mais non rectifié et contenant environ 1 500 ppm d'impuretés.

Un essai similaire aux essais 3 à 5 réalisé en l'absence d'initiateur n'a donné naissance à aucun produit de chloration.

TABLEAU I

| ESSAI | | 3 | 4 | 5 a | b |
|---|---|---|---|---|---|
| Réactifs | | | | | |
| CH$_3$Cl | g | 650 | 655 | 655 | |
| Cl$_2$ | g | 280 | 276 | 276 | |
| Cl$_2$:CH$_3$Cl | mole/mole | | | | |
| Catalyseur | | | | | |
| peroxyde de lauroyle, g | g | 0,8 | | | |
| 2,2'-azo-bis-2,4-diméthyl-valéronitrile | g | | 0,508 | 0,505 | |
| Solvant du catalyseur | | | | | |
| CCl$_4$ | g | 80,1 | 75,3 | 72,58 | |
| Température | °C | 45 | 45 | 45 | |
| Pression | kg/cm$^2$ | 11,0 | 12,7 | 12,6 | |
| Durée (t) | min | 180 | 60 | 3 | 60 |
| Produits obtenus | | | | | |
| CH$_2$Cl$_2$ | mole/l | 1,23 | 3,47 | 1,28 | 3,52 |
| CHCl$_3$ | mole/l | ~0,03 | 0,21 | ~0,02 | 0,21 |
| CCl$_4$* | mole/l | ~0 | ~0 | ~0 | ~0 |
| Sélectivité | mole/mole | | | | |
| CH$_2$Cl$_2$/CH$_3$Cl$_0$–CH$_3$Cl$_t$ | | 0,98 | 0,943 | 0,98 | 0,944 |
| CH$_2$Cl$_2$/(CHCl$_3$ + CCl$_4$) | | | 16,5 | | 16,8 |

*Valeur estimée par différence

L'examen des résultats présentés au Tableau I montre que le peroxyde de lauroyle et le 2,2-azo-bis-2,4-diméthylvaléronitrile permettent tous deux d'obtenir de bonnes sélectivités. Les vitesses réactionnelles sont cependant supérieures avec le composé diazo (comparaison des essais 3 et 5a).

La comparaison des essais 3 à 5 aux essais 1 et 2 montre que le procédé selon l'invention permet d'obtenir une meilleure sélectivité en dichlorométhane pour un même rapport chlore sur monochlorométhane.

## Revendications

1. Procédé pour la fabrication de dichlorométhane par chloration du monochlorométhane en phase liquide en présence d'un initiateur de réactions radicalaires caractérisé en ce qu'on opère substantiallement en l'absence de chlore gazeux et en présence d'un initiateur de réactions radicalaires choisi parmi les composés peroxydés et les composés diazo.

2. Procédé selon la revendication 1 caractérisé en ce que moins de 5% en poids du chlore mis en oeuvre se retrouve sous forme gazeuse dans le mélange réactionnel.

3. Procédé selon la revendication 1 ou 2 caractérisé en ce que l'initiateur de réactions radicalaires est choisi parmi ceux qui ont une constante de dissociation à 50°C dans le toluène comprise entre $1.10^{-6}$ et $2.10^{-4}$ sec$^{-1}$.

6

4. Procédé selon l'une quelconque des revendications 1 à 3 caractérisé en ce que l'initiateur de réactions radicalaires est un composé diazo.

5. Procédé selon la revendication 4 caractérisé en ce que le composé diazo est le 2,2'-azo-bis-2,4-diméthylvaléronitrile.

6. Procédé selon l'une quelconque des revendications 1 à 5 caractérisé en ce que la quantité d'initiateur mise en oeuvre est comprise entre $1.10^{-2}$ et $2.10^{-6}$ mole par mole de chlore mis en oeuvre.

7. Procédé selon l'une quelconque des revendications 1 à 6 caractérisé en ce que les quantités de chlore et de monochlorométhane mises en oeuvre sont telles que le rapport molaire chlore sur monochlorométhane est compris entre 0,1 et 0,5.

8. Procédé selon l'une quelconque des revendications 1 à 7 caractérisé en ce que l'on choisit une température comprise entre 15 et 80°C.

9. Procédé selon l'une quelconque des revendications 1 à 8 caractérisé en ce que l'on choisit une pression comprise entre 2 et 70 kg/cm$^{-2}$.

10. Procédé selon l'une quelconque des revendications 1 à 9 caractérisé en ce que la réaction est réalisée dans un réacteur de type tubulaire.


**Patentansprüche**

1. Verfahren zur Herstellung von Dichlormethan durch Chlorierung von Monochlormethan in flüssiger Phase in Anwesenheit eines Radikal-Reaktionsinitiators, dadurch gekennzeichnet, daß man im wesentlichen in Abwesenheit von gasförmigem Chlor arbeitet und in Anwesenheit eines Radikal-Reaktionsinitiators ausgewählt unter den Peroxidverbindungen und Diazoverbindungen.

2. Verfahren gemäß Anspruch 1, dadurch gekennzeichnet, daß weniger als 5 Gew.-% des eingesetzten Chlors sich in gasförmigem Zustand in der Reaktionsmischung befinden.

3. Verfahren gemäß Anspruch 1 oder 2, dadurch gekennzeichnet, daß der Radikal-Reaktionsinitiator ausgewählt ist unter solchen die eine Dissoziationskonstante bei 50°C in Toluol von $1.10^{-6}$ bis $2.10^{-4}$ sec$^{-1}$ besitzen.

4. Verfahren gemäß einem der Ansprüche i bis 3, dadurch gekennzeichnet, daß der Radikal-Reaktionsinitiator eine Diazoverbindung ist.

5. Verfahren gemäß Anspruch 4, dadurch gekennzeichnet, daß die Diazoverbindung 2,2'-Azo-bis-2,4-dimethylvaleronitril ist.

6. Verfahren gemäß einem der Ansprüche 1 bis 5, dadurch gekennzeichnet, daß die eingesetzte Initiatormenge $1.10^{-2}$ bis $2.10^{-6}$ Mol je Mol eingesetztes Chlor beträgt.

7. Verfahren gemäß einem der Ansprüche 1 bis 6, dadurch gekennzeichnet, daß die eingesetzten Chlor- und Monochlormethanmengen derart sind, daß das Molverhältnis von Chlor zu Monochlormethan zwischen 0,1 und 0,5 beträgt.

8. Verfahren gemäß einem der Ansprüche 1 bis 7, dadurch gekennzeichnet, daß man eine Temperatur zwischen 15 und 80°C wählt.

9. Verfahren gemäß einem der Ansprüche 1 bis 8, dadurch gekennzeichnet, daß man einen Druck zwischen 2 und 70 kg/cm² wählt.

10. Verfahren gemäß einem der Ansprüche 1 bis 9, dadurch gekennzeichnet, daß die Reaktion in einem Rohrreaktor durchgeführt wird.


**Claims**

1. Process for the manufacture of methylene chloride by the chlorination of methyl chloride in the liquid phase, in the presence of a free-radical initiator, characterised in that the reaction is carried out substantially in the absence of chlorine gas and in the presence of a free-radical initiator chosen from amongst peroxide compounds and diazo compounds.

2. Process according to Claim 1, characterised in that less than 5% by weight of the chlorine used is found again in the gaseous form in the reaction mixture.

3. Process according to Claim 1 or 2, characterised in that the free-radical initiator is chosen from amongst those having a dissociation constant of between $1.10^{-6}$ and $2.10^{-4}$ second$^{-1}$, at 50°C, in toluene.

4. Process according to any one of Claims 1 to 3, characterised in that the free-radical initiator is a diazo compound.

5. Process according to Claim 4, characterised in that the diazo compound is 2,2'-azo-bis-2,4-dimethylvaleronitrile.

6. Process according to any one of Claims 1 to 5, characterised in that the amount of initiator used is between $1.10^{-2}$ and $2.10^{-6}$ mol per mol of chlorine used.

7. Process according to any one of Claims 1 to 6, characterised in that the amount of chlorine and methyl chloride used are such that the molar ratio of chlorine to methyl chloride is between 0.1 and 0.5.

8. Process according to any one of Claims 1 to 7, characterised in that the temperature is chosen between 15 and 80°C.

9. Process according to any one of Claims 1 to 8, characterised in that the pressure is chosen between 2 and 70 kg/cm².

10. Process according to any one of Claims 1 to 9, characterised in that the reaction is carried out in a reactor of the tubular type.